# EUROPÄISCHE PATENTSCHRIFT

(11) **EP 2 525 225 B1**
(45) Veröffentlichungstag und Bekanntmachung des Hinweises auf die Patenterteilung: **15.08.2018**
(21) Anmeldenummer: 12003843.5
(22) Anmeldetag: 15.05.2012
(51) Int. Cl.: G01N 33/50, C12M 1/34, B01L 3/00

(54) **Vorrichtung und Verfahren zur Untersuchung der Differenzierung von Zellen**
Device and method for examining differentiation of cells
Installation et procédé d'analyse de la différentiation de cellules

(30) Priorität: 19.05.2011 DE 102011102071
(43) Veröffentlichungstag der Anmeldung: 21.11.2012
(73) Patentinhaber: Karlsruher Institut für Technologie, 76131 Karlsruhe (DE)
(72) Erfinder: Guber, Andreas, Prof. Dr., 76227 Karlsruhe (DE); Hermann, Dirk, 75217 Birkenfeld (DE); Schneider, Marc, 76356 Weingarten (DE); Ahrens, Ralf, Dr., 76149 Karlsruhe (DE); Kreppenhofer, Kristina, 76227 Karlsruhe (DE); Wedlich, Doris, Prof. Dr., 76227 Karlsruhe (DE); Kashef, Jubin, Dr., 76189 Karlsruhe (DE); Kim, Chorong, 68165 Mannheim (DE); Gradl, Dietmar, Dr., 76287 Rheinstetten (DE)

(56) Entgegenhaltungen:
- US-A1- 2006 154 361
- US-A1- 2006 160 066

## Beschreibung

Die Erfindung ist einerseits auf dem Gebiet der Zellbiologie, insbesondere der Stammzellbiologie, und andererseits auf dem Gebiet der Mikrofluidik angesiedelt und betrifft eine Vorrichtung und ein Verfahren zur Untersuchung der Differenzierung von Zellen, insbesondere von Stammzellen, bei Kontakt mit einem Gradienten einer flüssigen Lösung aus mindestens einer biologisch wirksamen Spezies, vorzugsweise Proteinen und/oder Morphogenen. Im Folgenden dient der Begriff *Protein* zusammenfassend als Bezeichnung sowohl für Proteine als auch für Morphogene.

Die Differenzierung von Stammzellen ist ein langwieriges Verfahren, das mehrere Wochen dauern kann und von der definierten Konzentration der Proteine in der Umgebung der Zelle abhängt. Als Vorrichtung hierfür werden üblicherweise Kaskadenmischer aus Polydimethylsiloxan (PDMS) eingesetzt, siehe insbesondere Stephan K. W. Dertinger, Daniel T. Chiu, Noo Li Jeon, und George M. Whitesides, Generation of Gradients Having Complex Shapes Using Microfluidic Networks, Anal. Chem. 73 (2001) 1240-1246; Francis Lin, Wajeeh Saadi, Seog Woo Rhee, Shur-Jen Wang, Sukant Mittal und Noo Li Jeon, Generation of dynamic temporal and spatial concentration gradients using microflui-dic devices, Lab Chip 4 (2004) 164-167; Thomas M. Keenan und Albert Folch, Biomolecular gradients in cell culture systems, Lab Chip 8 (2008) 34-57; Shur-Jen Wang, Wajeeh Saadi, Francis Lin, Connie Minh-Canh Nguyen, und Noo Li Jeon, Differential effects of EGF gradient profiles on MDA-MB-231 breast cancer cell chemotaxis, Experimental Cell Research 300 (2004) 180-189; Samuel K. Sia und George M. Whitesides, Microfluidic devices fabricated in poly(dimethylsiloxane) for biological studies, Electrophoresis 24 (2003) 3563-3576; und No Lee Jeon, Harihara Baskaran, Stephan K. W. Dertinger, George M. Whitesides, Livingston van De Water und Mehmet Toner, Neutrophil chemotaxis in linear and complex gradients of interleukin-8 formed in a microfabricated device, Nature Biotechnology 20 (2002) 826-830. Diesen Mischern ist gemeinsam, dass darin nur eines der beiden Bauteile strukturiert und das andere Bauteil unstrukturiert als Deckel aufgesetzt ist. Die Einzelströme werden nach der letzten Mischerkaskade vereint, und die Untersuchung der Differenzierung findet im vereinten Gradientenstrom statt, wobei sich durch einen separaten Zufluss Zellen auf Höhe des vereinten Gradientenstroms zugeben lassen. Nachteilig an dieser bekannten Art von Mischern ist, dass die Zellen hierin mechanischen Kräften ausgesetzt sind und die Untersuchungen im vereinten Gradientenstrom stattfinden, so dass eine definierte und zuverlässige Zuordnung von bestimmten Zellen zu bestimmten Mischungsverhältnissen nicht möglich ist.

Shin-ichiro Fujii, Munejisa Uematsu, Soichi Yabuki, Mitsuru Abo, Etsuro Yoshimura und Kiichi Sato, Microbioassay System for an Anticancer Agent Test Using Animal Cells on a Microfluidic Gradient Mixer, Analytical Sciences 22 (2006) 87-90, beschreiben einen Mikrofluidikchip aus PDMS mit einem strukturierten Bauteil, wobei zur Deckelung auf das strukturierte Bauteil ein nicht-strukturiertes Bauteil aus PDMS aufgesetzt wird. Zwei im rechten Winkel aufeinander treffenden Zuflüssen folgt ein Diffusionsmischungskanal, an dessen Ende sich eine Zellkammer mit acht integrierten Kanälen befindet. Am Ende der Zellkammer ist ein Ausfluss angebracht, der gleichzeitig als Zellzufluss eingesetzt wird. Dieser Mikrofluidikchip sieht vor, dass zunächst Zellen durch den Abfluss injiziert und in der Zellkammer kultiviert werden. Daraufhin werden zwei Lösungen in den Mischer injiziert und die Zellen kommen in Kontakt mit dem Gradienten. Nachteilig hieran ist, dass die Zellen dem Durchfluss durch den Mikrofluidikchip und damit mechanischen Kräften ausgesetzt sind. Zudem lassen sich die Zellen während der Laufzeit des Experiments nicht mit Nährlösung versorgen. Schließlich wird der Grad der Vermischung durch die Flussrate bestimmt.

Taesung Kim, Mikhail Pinelis und Michel M. Maharbiz, Generating steep, shear-free gradients of small molecules for cell culture, Biomed Microdevices 11 (2009) 65-73, beschreiben ein mikrofluidisches Bauteil aus PDMS, das drei Komponenten, d.h. ein Mischerteil und ein Zellteil, die durch eine Polyestermembran getrennt sind, aufweist. Die Zellen, die vor dem Versuch über das Mischerteil mit Nährlösung versorgt werden, werden in das Zellteil injiziert und adhärieren in einer Kammer auf der Membran. Das Mischerteil besteht aus einer Kammer mit zwei jeweils gegenüberliegend angeordneten Zuflüssen und Abflüssen. Durch Injektion von zwei Lösungen durch gegenüberliegende Zuflüsse bildet sich ein Gradient in der Kammer des Mischers aus. Über der Kammer befinden sich die Zellen, die durch den Gradienten beeinflusst werden. Damit ist es jedoch nicht möglich, einen definierten, zeitlich konstanten Gradienten in einem vom Mischer getrennten Bereich mit separaten Kanälen zu erzeugen; der Gradient bildet sich vielmehr nur in einem sehr schmalen Bereich an der Grenze der beiden Flüssigkeiten aus. Da die Vorrichtung keine abgetrennten Kanäle in der Zellkammer aufweist und daher die Zellen migrieren können, lässt sich eine Zelle keinem definierten Mischungsverhältnis der eingegebenen Lösungen zuordnen. Zudem können die Proteine in der Zellkammer lateral diffundieren, so dass einer Konfrontation jeder Zelle mit einer definierten Proteinmischung nicht sichergestellt ist. Es ist keine Versorgung der Zellen über den langen Zeitraum während der Versuchsdurchführung vorgesehen; die Zellen werden lediglich vor der Versuchsdurchführung über das Mischerteil mit Nährlösung versorgt.

Die WO 2010/025926 A1 offenbart eine Gastransfervorrichtung, umfassend mindestens zwei Kammern und mindestens eine gasdurchlässige und flüssigkeitsundurchlässige Membran, wobei die Kammern und die Membran voneinander getrennt sind und Membran auf mindestens einer Seite derart strukturiert ist, dass dadurch Kanäle und/oder Verästelungen auf der Membran gebildet werden. Nachteilig hieran ist, dass durch die Membran lediglich Gase, nicht keine Proteine hindurchgelangen können.

Die WO 2011/012995 A1 offenbart eine Vorrichtung zum Mischen und Austauschen von Fluiden mit einer ersten Kammer und einer hieran angrenzenden zweiten Kammer. Die erste Kammer ist hierbei eine von zwei Fluiden durchströmbare Mischkammer, während die zweite Kammer eine nur vom zweiten Fluid durchströmbare Fluidzuführ und -abführkammer ist. Zwischen den beiden Kammern befindet sich zumindest teilweise eine semipermeable Membran, die für Moleküle des zweiten Fluids, nicht jedoch des ersten Fluids durchlässig ist. Die Membran besteht aus einem Material oder ist damit beschichtet, zum dem die Moleküle eines der beiden Fluide eine geringere Affinität aufweisen. Nachteilig hieran ist, dass mit dieser Vorrichtung keine Bereitstellung eines Gradienten einer der beiden Lösungen möglich ist.

Ferner beschreibt US 2006/0154361 A1 eine Vorrichtung zur Aufzucht und Haltung von Zellen, bestehend aus Kammern, einer mindestens semipermeable Membran und Kanälen, welche mit Flüssigkeit durchströmbar sind. Der Aufbau erlaubt dabei den Austausch der Flüssigkeit mit den Zellen durch Diffusion, ohne dass es zur Zellmigration kommt. Die Vorrichtung ist auch zur Analyse von Zellen einsetzbar.

Ausgehend hiervon ist es die Aufgabe der vorliegenden Erfindung, eine Vorrichtung und ein Verfahren zur Untersuchung der Differenzierung von Zellen, insbesondere von Stammzellen, bei Kontakt mit einem Gradienten einer flüssigen Lösung aus mindestens einer biologisch wirksamen Spezies, insbesondere Proteinen und/oder Morphogenen, bereitzustellen, die die Nachteile und Einschränkungen des Stands der Technik überwinden.

Insbesondere soll eine Vorrichtung bereitgestellt werden, mit der es möglich wird, gleichzeitig den Einfluss von mehreren definierten Mischungsverhältnissen von flüssigen Lösungen aus biologisch wirksamen Spezies auf Zellen zu erforschen, wobei die Zellen während des langen Zeitraums der Versuchsdurchführung einerseits zuverlässig mit Nährlösung versorgt werden und andererseits keinen sich im Mischerteil ausbildenden mechanischen Kräften ausgesetzt sind.

Diese Aufgabe wird im Hinblick auf die Vorrichtung durch die Merkmale des Anspruchs 1, im Hinblick auf das Verfahren durch die Schritte des Anspruchs 7 gelöst. Die Unteransprüche beschreiben jeweils vorteilhafte Ausgestaltungen der Erfindung.

Eine erfindungsgemäße Vorrichtung zur Untersuchung der Differenzierung von Zellen bei Kontakt mit einem Gradienten einer flüssigen Lösung aus mindestens einer biologisch wirksamen Spezies enthält
- ein Kammerteil, das einen ersten Fluidikkreislauf zur Aufnahme von Zellen in Nährlösung besitzt, der eine Zellkammer aufweist, die in mindestens zwei Zellkanäle unterteilt ist,
- ein Mischerteil, das einen zweiten Fluidikkreislauf zur Aufnahme mindestens einer flüssigen Lösung, die mindestens eine biologisch wirksamen Spezies enthält, besitzt und der einen Mischer zur Erzeugung und Bereitstellung von mindestens drei unterschiedlichen Konzentrationen der mindestens einen Lösung in mindestens drei Kanälen aufweist, wobei die Anzahl der Kanäle im Mischerteil vorzugsweise gleich der Anzahl der Zellkanäle im Kammerteil ist, und
- eine Membran, die zwischen das Kammerteil und das Mischerteil angeordnet ist und die zumindest einen Perforationsbereich zum Inkontaktbringen der mindestens einen biologisch wirksamen Spezies in Lösung aus dem Mischerteil mit den Zellen in der Zellkammer besitzt, wobei der Perforationsbereich Poren aufweist, die einen Durchmesser von 0,05 µm bis 15 µm, bevorzugt 0,3 µm bis 1 µm besitzen.

Der Durchmesser der Poren ist hierbei so gewählt, dass die Membran sowohl flüssigkeits- als auch gasdurchlässig für die mindestens eine biologisch wirksame Spezies aus der Lösung ist, während sie eine Migration von Zellen aus dem Kammerteil durch die Membran in das Mischerteil verhindert.

Entscheidend für die besonders vorteilhafte Funktionsweise der erfindungsgemäßen Vorrichtung ist, dass die mindestens drei Zellkanäle zur Aufnahme der Zellen in Nährlösung in der Zellkammer in einem Reaktionsbereich jeweils formschlüssig über den mindestens drei Kanälen angeordnet sind, die im zweiten Fluidikkreislauf zur Aufnahme der mindestens drei unterschiedlichen Konzentrationen aus flüssigen, insbesondere wässrigen, Lösungen der mindestens einen biologisch wirksamen Spezies angeordnet sind.

In einer besonders bevorzugten Ausgestaltung sind die mindestens drei Kanäle zur Aufnahme der unterschiedlichen Konzentrationen der Lösung aus mindestens einer biologisch wirksamen Spezies flüssigkeitsdicht gegeneinander abgeschlossen.

In einer weiteren bevorzugten Ausgestaltung weisen sowohl die mindestens zwei Zellkanäle als auch die mindestens zwei Kanäle zur Aufnahme der unterschiedlichen Konzentrationen der Lösung aus mindestens einer biologisch wirksamen Spezies insbesondere im Reaktionsbereich überwiegend die Form einer geradlinigen Kanalstruktur auf.

In einer besonderen Ausgestaltung befinden sich sowohl die Anschlüsse des ersten Fluidikkreislaufs als auch die Anschlüsse (mindestens zwei Zuflüsse und mindestens ein Abfluss) des zweiten Fluidikkreislaufs im Mischerteil.

In einer alternativen Ausgestaltung sind die Anschlüsse des ersten Fluidikkreislaufs in das Kammerteil eingebracht, während sich die Anschlüsse (mindestens zwei Zuflüsse und mindestens ein Abfluss) des zweiten Fluidikkreislaufs im Mischerteil befinden.

Der Aufbau der erfindungsgemäßen Vorrichtung ermöglicht es, gleichzeitig den Einfluss von mehreren definierten Mischungsverhältnissen von Lösungen auf Zellen zu erforschen. Dies ist dadurch möglich, da sich jede Zelle durch die formschlüssige Verbindung der Kanäle in der Zellkammer und der vorzugsweise geradlinigen Kanäle des Mischerteils einem exakten Mischungsverhältnis der eingeleiteten Lösungen zuordnen lässt. Die Kanäle in der Zellkammer verhindern hierbei einerseits die Migration von Zellen zu anderen Konzentrationen und andererseits die laterale Diffusion von Proteinen in der Zellkammer. Die Strukturierung der Zellkammer mit Kanälen erhöht zusätzlich die Stabilität des Bauteils während der Herstellung mittels des Thermobondverfahrens.

Der Mischer erlaubt die Erzeugung eines vorzugsweise zeitlich konstanten und definierten Gradienten, der über die angrenzenden vorzugsweise überwiegend geradlinigen Kanäle im Reaktionsbereich zur Verfügung steht. Die mindestens teilweise perforierte Membran ist so ausgestaltet, dass lediglich im Reaktionsbereich eine Diffusion von Proteinen aus dem Mischerteil in das Kammerteil auftritt, und verhindert sie an allen anderen Stellen.

Die Zellen lassen sich aufgrund der Trennung von Mischer- und Kammerteil weitgehend unabhängig vom Mischerteil mit Nährlösung versorgen. Ebenso wenig sind die Zellen den sich im Mischerteil ausbildenden mechanischen Kräften ausgesetzt.

Der Mischer zur Erzeugung und Bereitstellung von mindestens drei unterschiedlichen Konzentrationen der mindestens einen Lösung in mindestens zwei Kanälen ist als Kaskadenmischer ausgestaltet. Das Mischungsverhältnis ist durch die detaillierte Ausgestaltung des Kaskadenmischers einstellbar, in Teilbereichen unabhängig von der Flussrate und lässt sich nach Dertinger et al. berechnen. Mittels des Kaskadenmischers bildet sich in den Kanälen des Mischerteils, die flüssigkeitsdicht gegeneinander abgeschlossen sind, ein definierter, vorzugsweise zeitlich konstanter Proteingradient aus.

In einer alternativen Ausführung lassen sich auch Mischerteile mit mehr als zwei Zuflüssen, wie z. B. in Dertinger et al. dargestellt, bereitstellen. Ebenso sind durch eine Erweiterung der Strukturen Kaskadenmischer mit weiteren Mischerstufen denkbar, wobei die jeweils gewählte Struktur der Kaskadenmischer die Anzahl der Kanäle im Mischerteil bedingt. Darüber hinaus sind auch Mischer einsetzbar, die nicht als Kaskadenmischer ausgestaltet sind.

Das Kammerteil und das Mischerteil werden durch Replikationstechniken hergestellt. Replikationsverfahren in Kunststoff sind im Vergleich zu anderen Herstellungsprozessen vergleichsweise kostengünstig.

Vorzugsweise werden diese Bauteile heißgeprägt, wobei auch die Herstellung im Spritzgussverfahren oder einer anderen Replikationstechnik möglich ist.

Das Thermobondverfahren ist besonders gut dazu geeignet, um Materialien mit nahe aneinander liegenden Glasübergangsbereichen zu verbinden. Die drei Komponenten des Mikrofluidikchips, d.h. Kammerteil, Mischerteil und Membran, werden daher bevorzugt mittels eines Thermobondverfahrens durch Druck und Temperatur verbunden.

Alternative Verbindungstechniken wie Laserschweißen, Lösungsmittelbonden, Ultraschallschweißen und der Einsatz von Klebstoffen oder eine Kombination der genannten Verfahren sind ebenfalls denkbar. Alternativ ist es möglich, die Mikrofluidikchips reversibel mechanisch zu verschließen, um sie am Ende des Versuchs öffnen zu können. Dies erlaubt Einsatz höherer Vergrößerungen des Mikroskops und damit eine verbesserte Analyse der Zellen im Anschluss an deren Behandlung. Vorteilhaft ist es hierbei, wenn eine weitere Schicht zur Abdichtung der mechanisch verschlossenen Bauteile eingefügt wird, um etwaige Leckagen zu vermeiden. Alternativ wird vorgeschlagen, einen reversiblen Verschlussmechanismus, wie z.B. einen Schnappverschluss, direkt in den Mikrofluidikchip zu integrieren.

Das für das Kammerteil und/oder das Mischerteil bevorzugt eingesetzte transparente Polycarbonat (PC) wirkt sich im Vergleich zu anderen auf dem Gebiet der Biologie eingesetzten Materialien vorteilhaft sowohl auf die Zelladhäsion als auch auf die Autofluoreszenz aus. Polycarbonate zeichnen sich durch eine sehr gute biologische Verträglichkeit und Beständigkeit gegen Wasser und Alkohol, mit Ausnahme von Methanol aus. Alternativ eignen sich andere biokompatible, vorzugsweise transparente Polymere, bevorzugt Polymethylmethacrylat (PMMA), Cycloolefincopolymer (COC) oder Polydimethylsiloxan (PDMS). Auch Glas oder der Einsatz einer Kombination der genannten Kunststoffe mit Gläsern ist möglich. Für die Membran eignet sich bevorzugt Polycarbonat (PC) oder ein anderes biokompatibles Material.

Das erfindungsgemäße Verfahren zur Untersuchung der Differenzierung von Zellen bei Kontakt mit Gradienten einer flüssigen Lösung aus mindestens einer biologisch wirksamen Spezies, umfasst die Schritte (a) bis (c).

Gemäß Schritt (a) werden Zellen in Nährlösung über einen Anschluss des Kammerteils in die Zellkammer eingebracht. Die Zellkammer ist in mindestens drei Zellkanäle unterteilt, die jeweils formschlüssig in einem Reaktionsbereich über den mindestens drei Kanälen des Mischerteils angeordnet sind. Die Zellen adhärieren an der zumindest teilweise perforierten Membran und lassen sich mit Nährlösung versorgen.

Gemäß Schritt (b) wird mindestens eine flüssige Lösung einer biologisch wirksamen Spezies in das Mischerteil (20) eingeführt und mindestens ein sich im Mischerteil befindlicher Mischer erzeugt unterschiedliche Konzentrationen der Lösung aus mindestens einer biologisch wirksamen Spezies. Die unterschiedlichen Konzentrationen der mindestens einen biologisch wirksamen Spezies in Lösung gelangen durch Poren eines Perforationsbereichs der Membran an die Zellen in den mindestens drei Zellkanälen des Kammerteils. Dadurch werden die Zellen in den mindestens drei Zellkanälen der betreffenden Konzentration der mindestens einen biologisch wirksamen Spezies aus der Lösung ausgesetzt.

Gemäß Schritt (c) werden die Zellen mittels eines optischen Verfahrens, insbesondere mittels eines Fluoreszenzmikroskops, einer Beobachtung unterzogen.

Die Erfindung wird im Folgenden anhand von Ausführungsbeispielen und den Figuren näher erläutert. Es zeigen im Einzelnen:
- **Fig. 1**: Querschnitt durch die erfindungsgemäße Vorrichtung;
- **Fig. 2**: Kammer- und Mischerteil in zwei unterschiedlichen Ausführungen: Die Anschlüsse beider Fluidikkreisläufe befinden sich in Ausführung **a)** im Mischerteil und in Ausführung **b)** im jeweiligen Bauteil;
- **Fig. 3**: **a)** Darstellung einer nur im Reaktionsbereich perforierten Membran und **b)** ihre Positionierung über dem Reaktionsbereich des Mischerteils.

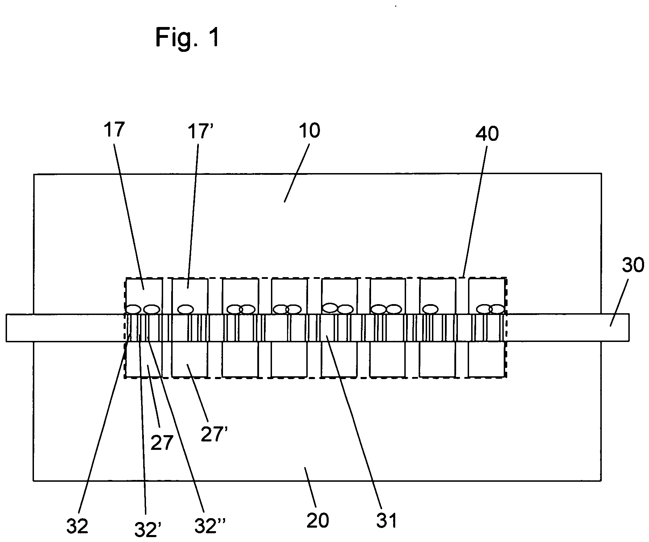

Die erfindungsgemäße Vorrichtung (Mikrofluidikchip) besteht aus zwei Kunststoffteilen, dem Kammerteil **10** und dem Mischerteil **20,** und einer zwischen den beiden Kunststoffteilen liegenden Membran **30,** die mindestens teilweise mit einem Perforationsbereich **31,** der Poren **32, 32', 32''...** aufweist, ausgestattet ist. Der Bereich, in dem jeweils acht Zellkanäle **17, 17'...** des Kammerteils **10** und acht Kanäle **27, 27'...** des Mischerteils **20** formschlüssig übereinander liegen, wird als Reaktionsbereich **40** bezeichnet. Ein Querschnitt durch den erfindungsgemäßen Mikrofluidikchip ist in **Fig. 1** dargestellt.

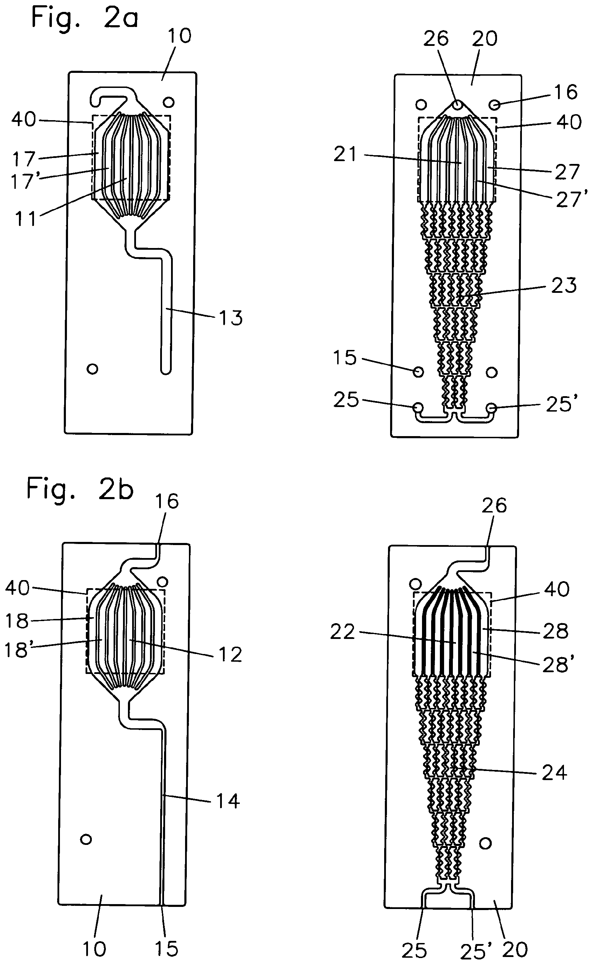

Der erfindungsgemäße Mikrofluidikchip besitzt zwei Fluidikkreisläufe, und zwar einen ersten Fluidikkreislauf zur Aufnahme von Zellen in Nährlösung, der sich im Kammerteil **10** befindet, sowie einen zweiten Fluidikkreislauf zur Aufnahme von Proteinlösungen, der im Mischerteil **20** vorgesehen ist. **Fig. 2** zeigt zwei unterschiedliche Ausführungen für die beiden Fluidikkreisläufe. Während in der Ausführung gemäß **Fig. 2a****)** die Anschlüsse **15, 16, 25, 25', 26** beider Fluidikkreisläufe in das Mischerteil **20** eingebracht sind, befinden sich in der Ausführung gemäß **Fig. 2b****)** die Anschlüsse **15, 16** des ersten Fluidikkreislaufs im Kammerteil **10** und die Anschlüsse **25, 25', 26,** des zweiten Fluidikkreislaufs im Mischerteil **20.**

Zwei flüssige Lösungen werden über Zuflüsse **25, 25'** in den zweiten Fluidikkreislauf eingeleitet, wobei mindestens eine der beiden Lösungen Proteine enthält. Die beiden Lösungen werden anschließend im Kaskadenmischer **23, 24** vermischt. Das Mischungsverhältnis ist durch die detaillierte Ausgestaltung des Kaskadenmischers **23, 24** einstellbar, in Teilbereichen unabhängig von der Flussrate und ließ sich nach Dertinger et al. berechnen. Mittels des Kaskadenmischers **23, 24** bildet sich in den anschließenden acht geradlinigen Kanälen **27, 27'..., 28, 28'...,** die flüssigkeitsdicht gegeneinander abgeschlossen sind, ein definierter, vorzugsweise zeitlich konstanter Proteingradient aus.

Die Zellen in Nährlösung werden bevorzugt über einen separaten Zufluss **15** in das Kammerteil **10** injiziert. Alternativ ist auch eine Injektion durch den Anschluss **16** geeignet. Sie werden bis in eine sechseckige Zellkammer **11, 12** geleitet, die in acht überwiegend geradlinige Zellkanäle **17, 17',** ..., **18, 18'...** unterteilt ist, die formschlüssig über den acht überwiegend geradlinigen Kanälen **27, 27',** ..., **28, 28',** ..., des Mischerteils **20** angeordnet sind. Die Zellen adhärieren auf der zumindest teilweise perforierten Membran **30** und lassen sich so über den Zellkreislauf über mehrere Wochen hinweg mit Nährlösung versorgen.

Die Proteine aus der im Mischerteil **20** enthaltenen Lösung diffundieren durch Poren **32, 32', 32''** ... im Perforationsbereich **31** der mindestens teilweise perforierten Membran **30** an die Zellen in den Zellkanälen **17, 17',** ..., **18, 18',** ..., des Kammerteils **10** und gelangen dadurch unmittelbar in die Umgebung der Zellen. Der Bereich, in dem die jeweils acht Zellkanäle **17, 17',** ..., **18, 18',** ..., des Kammerteils **10** und die acht Kanäle **27, 27',** ..., **28, 28',** ..., des Mischerteils **20** formschlüssig übereinander liegen, wird als Reaktionsbereich **40** bezeichnet und ist schematisch in den **Fig. 2a) und 2b****)** dargestellt. Die Zellen im Mikrofluidikchip lassen sich mittels eines Fluoreszenzmikroskops beobachten.

Die erfindungsgemäße Anordnung stellt sicher, dass jede Zelle ausschließlich mit der zugehörigen Proteinkonzentration aus dem jeweils darunter liegenden geradlinigen Kanal **27, 27',** ..., **28, 28',** ..., des Mischerteils **20** in Kontakt gelangt, d.h. Zellen, die sich im Zellkanal **17, 18** befinden, treten ausschließlich mit der, eine definierten Konzentration aufweisende Proteinlösung in Kontakt, die den geradlinigen Kanal **27, 28** im Mischerteil **20** durchströmt, usw. Gleichzeitig wird sichergestellt, dass ein bestimmtes Proteingemisch und die jeweils zugeordneten Zellen räumlich von allen anderen Proteingemischen und jeweils zugeordneten Zellen getrennt sind.

Das Kammerteil **10** und das Mischerteil **20** wurden durch Heißprägetechnik hergestellt. Als Material für das Kammerteil **10** und das Mischerteil **20** diente besonders bevorzugt transparentes Polycarbonat (PC). Die Polycarbonat-Oberfläche des Mikrofluidikchips wurde mindestens teilweise glasklar poliert, um die Beobachtung mit dem Fluoreszenzmikroskop zu ermöglichen.

Als Membran **30** diente eine über den Perforationsbereich **31** ionenspurgeätzte Membran aus Polycarbonat (PC), deren Poren **32, 32', 32'',** ... einen Durchmesser von 0,4 µm aufweisen.

Die drei Komponenten des Mikrofluidikchips, d.h. Kammerteil **10,** Mischerteil **20** und Membran **30,** wurden mittels eines Thermobondverfahrens durch Druck und Temperatur verbunden.

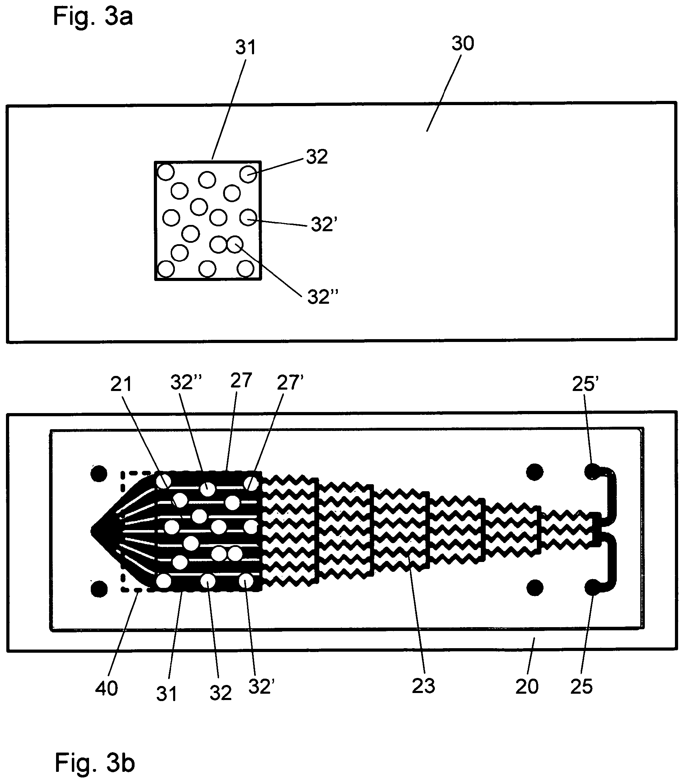

Der Perforationsbereich **31** der mindestens teilweise perforierten Membran **30** muss, wie in **Fig. 3a****)** dargestellt, den Reaktionsbereich **40** zumindest teilweise überdecken. Bei Einsatz einer auch außerhalb des Reaktionsbereichs **40** perforierten Membran **30** ist es ggf. erforderlich, den Aufbau des Kammerteils **10** und/oder des Mischerteils **20** so zu verändern, dass lediglich im Reaktionsbereich **40** Diffusion aus dem Mischerteil **20** in das Kammerteil **10** erfolgen kann.

### Liste der Bezugszeichen

- **10**: Kammerteil
- **11,12**: Zellkammer mit Zellkanälen **17,17',18,18'**
- **13,14**: Kanäle des Kammerteils
- **15**: erster Anschluss (bevorzugt Zufluss) für Zellen in Nährlösung
- **16**: zweiter Anschluss (bevorzugt Abfluss) für Zellen in Nährlösung
- **17,17',18,18'**: Zellkanäle

- **20**: Mischerteil
- **21,22**: Bereich, in dem die Kanäle **27,27',28,28'** des Mischerteils **20** ausgebildet sind
- **23,24**: Kaskadenmischer
- **25,25'**: Zuflüsse (Anschlüsse) für flüssige Lösung mit biologisch wirksamer Spezies
- **26**: Abfluss (Anschluss) für flüssige Lösung mit biologisch wirksamer Spezies
- **27,27',28,28'**: Kanäle des Mischerteils **20**

- **30**: Membran
- **31**: Perforationsbereich der Membran **30**
- **32, 32', 32''...**: Poren im Perforationsbereich **31** der Membran **30**
- **40**: Reaktionsbereich

## Patentansprüche

1. Vorrichtung zur Untersuchung der Differenzierung von Zellen bei Kontakt mit einem Gradienten einer flüssigen Lösung aus mindestens einer biologisch wirksamen Spezies, **umfassend**
- ein Kammerteil (10) mit einem ersten Fluidikkreislauf zur Aufnahme von Zellen in Nährlösung,
- ein Mischerteil (20) mit einem zweiten Fluidikkreislauf zur Aufnahme einer flüssigen Lösung aus mindestens einer biologisch wirksamen Spezies und einem Kaskadenmischer (23, 24) zur Bereitstellung von mindestens drei unterschiedlichen Konzentrationen der Lösung in mindestens drei Kanälen (27, 27', 28, 28'),
- wobei der erste Fluidikkreislauf eine Zellkammer (11, 12) aufweist, die in mindestens zwei Zellkanäle (17, 17', 18, 18') zur Aufnahme der Zellen in Nährlösung unterteilt ist, die in einem Reaktionsbereich (40) jeweils formschlüssig über den mindestens zwei Kanälen (27, 27', 28, 28') im Mischerteil (20) angeordnet sind, und
- eine Membran (30), die zwischen das Kammerteil (10) und das Mischerteil (20) eingebracht ist und die im Reaktionsbereich (40) zum Inkontaktbringen der mindestens einen biologisch wirksamen Spezies in der Lösung aus dem Mischerteil (20) mit den Zellen in der Zellkammer (11) zumindest teilweise perforiert ist, wobei der Perforationsbereich (40) Poren aufweist, die einen Durchmesser von 0,05 µm bis
15 µm besitzen.

2. Vorrichtung nach Anspruch 1, wobei die mindestens drei Kanäle (27, 27', 28, 28') zur Aufnahme der unterschiedlichen Konzentrationen der Lösung flüssigkeitsdicht gegeneinander abgeschlossen sind.

3. Vorrichtung nach einem der Ansprüche 1 oder 2, wobei der erste Fluidikkreislauf einen ersten Anschluss (15) und einen zweiten Anschluss (16) aufweist und der zweite Fluidikkreislauf mindestens zwei Zuflüsse (25, 25') und mindestens einen Abfluss (26) aufweist, wobei die Anschlüsse (15, 16), die mindestens zwei Zuflüsse (25, 25') und der mindestens eine Abfluss (26) in das Mischerteil (20) eingebracht sind.

4. Vorrichtung nach einem der Ansprüche 1 oder 2, wobei der erste Fluidikkreislauf einen ersten Anschluss (15) und einen zweiten Anschluss (16) aufweist, wobei die Anschlüsse (15, 16), in das Kammerteil (10) eingebracht sind, und der zweite Fluidikkreislauf mindestens zwei Zuflüsse (25, 25') und mindestens einen Abfluss (26) aufweist, wobei die mindestens zwei Zuflüsse (25, 25') und der mindestens eine Abfluss (26) in das Mischerteil (20) eingebracht sind.

5. Vorrichtung nach einem der Ansprüche 1 bis 4, wobei das Kammerteil (10) und/oder das Mischerteil (20) Polycarbonat (PC) oder ein anderes biokompatibles Polymer, bevorzugt Polymethylmethacrylat (PMMA), Cycloolefincopolymer (COC) oder Polydimethylsiloxan (PDMS) enthalten.

6. Vorrichtung nach einem der Ansprüche 1 bis 5, wobei die Membran (30) Polycarbonat (PC) oder ein anderes biokompatibles Material enthält.

7. Verfahren zur Untersuchung der Differenzierung von Zellen bei Kontakt mit einem Gradienten einer flüssigen Lösung aus mindestens einer biologisch wirksamen Spezies,
**mit den Schritten**
(a) Einbringen von Zellen in Nährlösung über einen Anschluss (15, 16) eines Kammerteils (10) in eine Zellkammer (11, 12), die in mindestens zwei Zellkanäle (17, 17', 18, 18') unterteilt ist, die formschlüssig in einem Reaktionsbereich (40) über mindestens drei Kanälen (27, 27', 28, 28') eines Mischerteils (20) angeordnet sind, wodurch die Zellen an einer zumindest teilweise perforierten Membran (30) adhärieren und sich mit Nährlösung versorgen lassen;
(b) Zuführen einer flüssigen Lösung aus mindestens einer biologisch wirksamen Spezies in das Mischerteil (20), Erzeugen von unterschiedlichen Konzentrationen der Lösung mittels eines in das Mischerteil (20) eingebrachten Kaskadenmischers (23, 24) und Diffundierenlassen der unterschiedlichen Konzentrationen der Lösung durch Poren (32, 32', 32''...) eines Perforationsbereichs (31) der Membran (30) an die Zellen in den mindestens zwei Zellkanälen (17, 17', 18, 18') des Kammerteils (10), wodurch die Zellen der mindestens zwei Zellkanäle (17, 17', 18, 18') der jeweiligen Konzentration an der mindestens einen biologisch wirksamen Spezies in der Lösung ausgesetzt werden; und
(c) Beobachten der Zellen mittels eines optischen Verfahrens.

8. Verfahren nach Anspruch 7, wobei das Beobachten der Zellen mittels eines Fluoreszenzmikroskops erfolgt.

9. Verfahren nach Anspruch 7 oder 8, wobei Proteine und/oder Morphogene als die mindestens eine biologisch wirksame Spezies eingesetzt werden.

## Claims

1. Device for examining the differentiation of cells on contact with a gradient of a fluid solution of at least one biologically effective species, **comprising**
- a chamber part (10) with a first fluid circuit for accommodating cells in nutrient solution,
- a mixer part (20) with a second fluid circuit for accommodating a fluid solution of at least one biologically effective species and a cascade mixer (23, 24) for providing at least three different concentrations of the solution in at least three channels (27, 27', 28, 28'),
- wherein the first fluid circuit comprises a cell chamber (11, 12), which is subdivided into at least two cell channels (17, 17', 18, 18') for accommodating the cells in nutrient solution, which in a reaction region (40) are arranged in each case in the mixer part (20) in positive fit connection by way of the at least two channels (27, 27', 28, 28'), and
- a membrane (30), which is introduced between the chamber part (10) and the mixer part (20) and which is at least partially perforated in the reaction region (40) for bringing the at least one biologically effective species in the solution from the mixer part (20) into contact with the cells in the cell chamber (11), wherein the perforation region (40) exhibits pores which have a diameter of 0.05 µm to 15 µm.

2. Device according to claim 1, wherein the at least three channels (27, 27', 28, 28') for accommodating the different concentrations of the solution are closed so as to be fluid-tight in relation to one another.

3. Device according to claim 1 or 2, wherein the first fluid circuit comprises a first connection (15) and a second connection (16), and the second fluid connection comprises at least two inflows (25, 25') and at least one outflow (26), wherein the connections (15, 16), the at least two inflows (25, 25'), and the at least one outflow (26) are introduced into the mixer part (20).

4. Device according to claim 1 or 2, wherein the first fluid circuit comprises a first connection (15) and a second connection (16), wherein the connections (15, 16) are introduced into the chamber part (10), and the second fluid circuit comprises at least two inflows (25, 25') and at least one outflow (26), wherein the at least two inflows (25, 25') and the at least one outflow (26) are introduced into the mixer part (20).

5. Device according to any one of claims 1 to 4, wherein the chamber part (10) and/or the mixer part (20) contains polycarbonate (PC) or another biocompatible polymer, preferably polymethyl methacrylate (PMMA), cyclo olefin copolymer (COC), or polydimethyl siloxane (PDMS).

6. Device according to any one of claims 1 to 5, wherein the membrane (30) contains polycarbonate (PC) or another biocompatible material.

7. Method examining the differentiation of cells on contact with a gradient of a fluid solution of at least one biologically effective species,
**comprising the steps**
(a) introduction of cells in nutrient solution by way of a connection (15, 16) of a chamber part (10) into a cell chamber (11, 12), which is subdivided into at least two cell channels (17, 17', 18, 18'), which are arranged in positive fit connection in a reaction region (40) by way of at least three channels (27, 27', 28, 28') of a mixer part (20), as a result of which the cells adhere to an at least partially perforated membrane (30) and can be supplied with nutrient solution;
(b) delivery of a fluid solution of at least one biologically effective species into the mixer part (20), production of different concentrations of the solution by means of a cascade mixer (23, 24) introduced into the mixer part (20), and diffusing of the different concentrations of the solution through pores (32, 32', 32" ...) of a perforation region (31) of the membrane (30) to the cells in the at least two cell channels (17, 17', 18, 18') of the chamber part (10), as a result of which the cells of the at least two cell channels (17, 17', 18, 18') of the respective concentration are subjected to the at least one biologically effective species in the solution; and
(c) observation of the cells by means of an optical method.

8. Method according to claim 7, wherein the observation of the cells takes place by means of a fluorescence microscope.

9. Method according to claim 7 or 8, wherein proteins and/or morphogens are used as the at least one biologically effective species.

## Revendications

1. Dispositif d'analyse de la différentiation de cellules par contact avec un gradient d'une solution liquide d'au moins une espèce biologiquement active comprenant :
- une partie de chambre (10) comprenant un premier circuit fluidique permettant de recueillir des cellules dans une solution nutritive,
- une partie de mélange (20) comprenant un second circuit fluidique permettant de recueillir une solution liquide d'au moins une espèce biologiquement active et un mélangeur en cascade (23, 24) permettant d'obtenir au moins trois concentrations différentes de la solution dans au moins trois canaux (27, 27', 28, 28'),
- le premier circuit fluidique comprenant une chambre de cellules (11, 12) qui est subdivisée en au moins deux canaux de cellules (17, 17', 18, 18') permettant de recueillir les cellules dans la solution nutritive et qui sont respectivement situés dans la partie de mélange (20) dans une zone réactionnelle (40) positionnée par une liaison par la forme au dessus des canaux (27, 27', 28, 28'), et
- une membrane (30) qui est insérée entre la partie de chambre (10) et la partie de mélange (20) et qui est au moins partiellement perforée dans la zone réactionnelle (40) pour permettre la mise en contact de l'espèce biologiquement active de la solution sortant de la partie de mélange (20) avec les cellules dans la chambre de cellules (11), la zone perforée (40) ayant des pores ayant un diamètre de 0,05 µm à 15 µm.

2. Dispositif conforme à la revendication 1, dans lequel les canaux (27, 27', 28, 28') permettant de recueillir les différentes concentrations de la solution qui sont au moins au nombre de trois sont fermés les uns par rapport aux autres de façon étanche aux liquides.

3. Dispositif conforme à l'une des revendications 1 et 2, dans lequel le premier circuit fluidique comporte un premier raccord (15) et un second raccord (16) et le second circuit fluidique comporte au moins deux entrées (25, 25') et au moins une sortie (26), les raccords (15, 16), les entrées (25, 25') et la sortie (26) étant situés dans la partie de mélange (20).

4. Dispositif conforme à l'une des revendications 1 et 2, dans lequel le premier circuit fluidique comporte un premier raccord (15) et un second raccord (16), les raccords (15, 16) étant situés dans la partie de chambre (10), et le second circuit fluidique comporte au moins deux entrées (25, 25') et au moins une sortie (26), les deux entrées (25, 25') et la sortie (26) étant situées dans la partie de mélange (20).

5. Dispositif conforme à l'une des revendications 1 à 4, dans lequel la partie de chambre (10) et/ou la partie de mélange (20) renferme(nt) un polycarbonate (PC) ou un autre polymère biocompatible, de préférence du polyméthylméthacrylate (PMMA), un copolymère de cyclo-oléfines (COC) ou du polydiméthylsiloxane (PDMS).

6. Dispositif conforme à l'une des revendications 1, à 5, dans lequel la membrane (30) renferme du polycarbonate (PC) ou un autre matériau biocompatible.

7. Procédé permettant d'analyser la différentiation de cellules par contact avec un gradient d'une solution liquide d'au moins une espèce biologiquement active, comprenant des étapes consistant à :
(a) introduire des cellules dans une solution nutritive par un raccord (15, 16) d'une partie de chambre (10) dans une chambre de cellules (11, 12) qui est subdivisée en au moins deux canaux de cellules (17, 17', 18, 18') qui sont positionnés par une liaison par la forme dans une zone réactionnelle (40) située au dessus au moins trois canaux (27, 27', 28, 28') d'une partie de mélange (20) de sorte que les cellules adhèrent sur une membrane (30) au moins partiellement perforée et puissent être alimentées en solution nutritive,
(b) introduire une solution liquide d'au moins une espèce biologiquement active dans la partie de mélange (20), obtenir différentes concentrations de la solution au moyen d'un mélangeur en cascade (23, 24) inséré dans la partie de mélange (20) et faire diffuser les différentes concentrations de la solution au travers de pores (32, 32', 32") d'une zone perforée (31) de la membrane (30), sur les cellules des deux canaux de cellules (17, 17', 18, 18') de la partie de chambre (10), de sorte que les cellules des deux canaux de cellules (17, 17', 18, 18') ayant la concentration respective soient exposées à l'espèce biologiquement active dans la solution, et
(c) examiner les cellules par un procédé optique.

8. Procédé conforme à la revendication 7,
selon lequel,
l'observation des cellules est effectuée au moyen d'un microscope à fluorescence.

9. Procédé conforme à la revendication 7 ou 8,
selon lequel,
des protéines et/ou des morphogènes sont mis en oeuvre en tant qu'espèce biologiquement active.
